# EUROPEAN PATENT APPLICATION

(11) **EP 3 933 589 A1**
(43) Date of publication of application: **05.01.2022**
(21) Application number: 20183295.3
(22) Date of filing: 30.06.2020
(51) Int. Cl.: G06F 9/54, G16H 10/40, G16H 40/63, G16H 40/67, G05B 23/02

(54) **AN EVENT CHAIN REACTION SYSTEM**

(71) Applicant: Roche Diagnostics GmbH, 68305 Mannheim (DE); F. Hoffmann-La Roche AG, 4070 Basel (CH)
(72) Inventor: Jiménez Roda, Javier, 6343 Rotkreuz (CH); Mazpule Elizalde, Julene, 6343 Rotkreuz (CH)
(74) Representative: Altmann Stößel Dick Patentanwälte PartG mbB

(57) **Abstract**

An event chain reaction system (128) is disclosed. The event chain reaction system (128) comprises:
- at least one communication interface (156) configured for receiving at least one event stream (160), wherein the event stream (160) comprises at least one sequence of ordered events generated by at least one analytical system (112), wherein each event comprises information about a change in a state of the analytical system (112) and/or any of loaded resources;
- at least one chain reaction component (158) comprising at least one chain matching element (162), wherein the chain matching element (162) is configured for recognizing at least one chain on the event stream (160), wherein the chain comprises a set of ordered events to be searched, wherein a first event of the chain defines a start event (166), wherein the chain matching element (162) is configured for identifying the start event (166) in the event stream (160) and, upon identifying the start event (166), the chain matching element (162) is configured for successively determining whether the other events of the chain match to one of the events of the event stream (160), wherein, in case all events of the chain are matched to events of the event stream (160), the chain matching element (162) is configured for triggering at least one reaction, wherein the reaction comprises generating information that a chain was matched and/or issuing a command to at least one component of the analytical system (112), wherein, in case one of the events of the chain is not matched, the chain matching element (162) is configured for resetting to its initial state and waiting for the start event (166).

Further, a system (110) for monitoring and/or controlling, a computer implemented method and a computer program for determining at least one feature of at least one component of an analytical system (112), a computer implemented method and a computer program for monitoring and/or controlling at least one feature of at least one component of an analytical system (112) are disclosed.

## Description

### Technical Field

The invention relates to an event chain reaction system and to a system, a computer-implemented method and a computer program for monitoring and/or controlling at least one feature of at least one component of an analytical system. The invention further relates to a computer-implemented method and a computer program for determining the at least one feature of the at least one component of the analytical system. The methods and devices of the present invention, as an example, may be used in the field of data analytics, specifically for processing and/or monitoring event streams, such as event streams produced in an analytical system. Other fields of application are, however, also feasible.

### Background art

In the field of analytical systems, for example analytical system including analyzers, pre-post processors and/or point of care device, in many cases, analytical system may be capable of producing a set of events during operation. These events may represent changes in the state of the analytical system which are produced by facts. Events may comprise information about a state of the analytical system and/or the components of the analytical system. Examples of such events may be sample rack loaded, over temperature detected, fuse broken, or system state changed. Every event may provide details in the shape of its payload. For example, an event describing that a sample was loaded to the analytical system may comprise, as payload, the rack barcode, the slot in the instrument where it was loaded and/or barcodes of each of the tube comprised in the rack. The event may also comprise a time stamp when the event was produced and/or an originator identifier indicating which identity has produced the event.

In general, events provide also an explanation of behavioral aspects of the execution of the functionality of the analytical system. Events may be related with each other, thereby building a history which tells what the analytical system was doing at a certain point in time or how the analytical system went into a given state. Further, events may not only provide details about what happened but also may explain the behavioral aspects behind them.

With respect to data analytics of these events, having a set of events as source of data may be extremely useful because it may provide different perspectives of the analytical system. On the one hand, the pure data perspective may be of specific interest. E.g. how many samples a system is able to process or how often customers load racks. On the other hand, the events may provide a behavioral perspective which, in general, may be obtained with the pure data only. For example, when does customers run HIV tests, how many runs are needed till the fuse breaks, how many samples are usually loaded Mondays at 10 o'clock. These set of events may be seen as an event stream.

In general, it is an objective to analyze the event stream by searching for certain information. Further, it may be of interest to detect behaviors by looking at the event stream, such as by performing pattern matching on the event stream.

US 2015/0227838 A1 discloses a method of building a model for predicting failure of a machine, including parsing daily machine event logs of one or more machines to extract data for a plurality of features, parsing service notifications for the one or more machine to extract failure information data, creating bags from the daily machine event log data and failure information data for multiple instance learning by grouping daily event log data into the bags based on a predetermined predictive interval, labeling each bag with a with a known failure as positive, and bags without known failures as negative, where a bag is a set of feature vectors and an associated label, where each feature vector is an n-tuple of features, transforming the multiple instance learning bags into a standard classification task form, selecting a subset of features from the plurality of features, and training a failure prediction model using the selected subset of features.

US 2010/0005342 A1 discloses a clinical diagnostic analyzer employing a redundant error detection capability to further examine the internal message traffic for possible errors or questionable results by comparing the actual parameters against a fingerprint generated for each assay using an assay database and a configuration file. This testing does not rely on inputs from the software module being tested, and hence is an independent test. Further, a testing mechanism is provided to test the Redundant Error Detection ("RED") capability itself.

US 2013/0132001 A1 discloses a tool and method relate to device fault detection, diagnosis and prognosis. More particularly, the tool and method store in a database a plurality of measured indicators representative of at least one dynamic condition of the device. The tool and method further binarized by a processor the plurality of measured indicators, and analyze the plurality of binarized measured indicators using a machine learning data tool for extracting at least one pattern from the binarized measured indicators by adding at least one different constraint to each iteration. The at least one extracted pattern is indicative of whether the device has a fault or not.

WO 2012/037429 A2 discloses a method of failure prediction in a work cycle includes generating a plurality of rules for predicting failure, evaluating the plurality of rules for predictability with respect to at least one of a machine and a message set generated by the machine and a sensor generating sensor data, ranking the plurality of rules based on the evaluation, and refining at least one rule of the plurality of rules having a threshold ranking.

Despite the advantages achieved by the known methods and devices several technical challenges remain. Specifically, flexibility in terms of detecting issues or trends in the event stream needs to be enhanced and ensured. In general, detecting a mutated sequence of events remains a major technical challenge. Further, another technical challenge may refer to the possibility of moving from a reactive to a predictive solution, such that the analysis may predict what the user of the analytical system is going to do next.

### Problem to be solved

It is therefore desirable to provide methods and devices which at least partially address the above-mentioned technical challenges. Specifically it is desirable to provide methods and devices which improve the flexibility of analyzing events of an analytical system and further improve the usability and the performance of the analytical system.

### Summary

This problem is addressed by methods, devices and computer programs an event chain reaction system, a system for monitoring and/or controlling comprising said event chain reaction system, a computer implemented method and a computer program for determining and a computer implemented method and a computer program for monitoring and/or controlling at least one feature of at least one component of an analytical system with the features of the independent claims. Advantageous embodiments which might be realized in an isolated fashion or in any arbitrary combinations are listed in the dependent claims as well as throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

In a first aspect of the invention, an event chain reaction system is disclosed. The term "event" as used herein is a broad term and is to be given its ordinary and customary meaning to a person of ordinary skill in the art and is not to be limited to a special or customized meaning. The term specifically may refer, without limitation, to an arbitrary item of information representing a change of a state of an arbitrary system and/or any component thereof and/or any of loaded resources. The event may be produced by one or more facts, such as by external and/or internal factors influencing the state of the system. Specifically, the event may refer to a change of a state of an analytical system and/or any part thereof. The loaded resource may refer to any object or component handled by the analytical system, for example a sample rack or a sample container. Events may be written in past sense to express the fact that something happened and/or something changed. The event may describe a fact that happened and/or may provide insights on what happened in its payload.

The analytical system may comprise and/or can be decomposed into multiple hardware and software components. Each of these components may be stateful such that they keep their current state. For example: a hardware component can be in state "Ready", "Error", "Processing", etc. A software component can be, for example, a work order. The work order can be in state "Scheduled", "Processing", "Flagged", "Finished", "Aborted" and the like. Hardware components and software components may be configured for generating events that represent changes in the state of the system or any of its components. Hardware is, by nature, event-driven. E.g. sensors may generate signals based on physical interactions. Sensor signals may be one type of events. The term "change of state" as used herein is a broad term and is to be given its ordinary and customary meaning to a person of ordinary skill in the art and is not to be limited to a special or customized meaning. The term specifically may refer, without limitation, to changing of a state of at least one component of the analytical system at functional level, like the previous examples of state, and/or at behavioral level. For example, the behavioral state may refer to one or more of: user logged in, user started a run, sample aspirated, sample dispensed or the like.

The analytical system may comprise at least one sensor which may be configured for sensing the state of the analytical system and/or any parts thereof, and further for producing a signal indicating the change of state. The event may be an event of a specified type. For example, the event may comprise information such as sample rack loaded, over-temperature detected, fuse broken or system state changed. The type of the event may be specified with respect to information comprised by the event. For example types of such events may be "sample rack loaded", "over temperature detected", "fuse broken", and "system state changed".

The event further comprises payload, also denoted payload data. The term "payload" as used herein is a broad term and is to be given its ordinary and customary meaning to a person of ordinary skill in the art and is not to be limited to a special or customized meaning. The term specifically may refer, without limitation, to one or more item of information associated with the event. For example, the event may comprise at least one time stamp when the event was produced. The time stamp may indicate a point in time at which the event occurred. The event may comprise at least one originator identifier indicating which identity has produced the event. The originator identifier may identify the system and/or the part thereof where the event was recorded. For example, the originator identifier may be a slot and/or a position number indicating the slot or position at which a sample rack or a single sample was loaded to the analytical system. The payload of an event may also comprise a barcode, specifically a barcode of a sample rack and/or a barcode of a sample contained by the sample rack.

For example, the analytical system may comprise a sensor that detects that a rack was loaded and sends a signal indicating that. After this, the analytical system may be configured for scanning a barcode of each sample tube in the rack. Thus, in this example, the events and their payload may comprise the information "rack loaded" including the point in time and the slot of the analytical system into which the rack was loaded, and further the rack's barcode. For example, the event "rack loaded" may comprise in the payload information about the rack's barcode, the timestamp indicating the time at which the rack was loaded to the analytical system and the slot number of the analytical system.

For example, the analytical system may comprise a sensor that detects and identifies a sample container and sends a signal indicating that. The event may be published referring to the sample container comprised by the rack, which may be published once for every sample container, together with a timestamp, a position of the sample container in the rack and the sample container's barcode. For example, the event "sample container identified" may comprise in the payload information about the sample container's barcode, the timestamp and the position in the rack. The event "sample container identified" may be published once for every identified sample container.

The term "event chain", also referred to as "chain", as used herein is a broad term and is to be given its ordinary and customary meaning to a person of ordinary skill in the art and is not to be limited to a special or customized meaning. The term specifically may refer, without limitation, to an ordered set of events. In particular, an event chain may refer to a set of events that needs to be searched for. The event chain may be or may comprise an ordered set of events that were produced by a system, for example by an analytical system, while executing a certain operation. Specifically, the events comprised by the event chain may be ordered with respect to their time stamp. Thus, the event chain may be generated during operation of the system. Further, the event chain may comprise a finite number of events. Thus, events comprised by the event chain may be ordered. The events may be ordered based on the point in time they were produced, which may be indicated by the time stamp. Events produced in the exactly same time may be a rare exception and, in that case, this means that components that react to one of the events are usually not reacting to the other one.

The chain may specifically be an ordered set of events indicative for a feature of the analytical system, such as an item of information indicating a change of state of the analytical system. The term "feature", as used herein, is a broad term and is to be given its ordinary and customary meaning to a person of ordinary skill in the art and is not to be limited to a special or customized meaning. The term specifically may refer, without limitation, to a known and/or detectable issue on the analytical system. The chain of specific events may be created to detect the feature, so to be able to detect the issue, wherein the issue can be at hardware or software level or a combination of both. The information about a change in a state of the analytical system may be at least one of an operational information or a sensor signal generated by at least one sensor of the analytical system. The information about a change in a state of the analytical system may be at least one information selected from the group consisting of: a sensor signal indicating that performance is changing; a sensor signal indicating that a sample rack is loaded; a sensor signal indicating that sample container is identified, a sensor signal indicating that over temperature is detected, a sensor signal indicating that a fuse is broken, a sensor signal indicating that performance is changing, a sensor signal indicating that at least one hardware component of the analytical system performs differently as expected.

The term "reaction" as used herein is a broad term and is to be given its ordinary and customary meaning to a person of ordinary skill in the art and is not to be limited to a special or customized meaning. The term specifically may refer, without limitation, to an action in response to a certain occurrence. For example, the reaction may be one or more of producing and/or publishing an event that specifies that an event chain was matched and/or issuing a command to one or more different systems to react to the match.

The term "producing" as used herein is a broad term and is to be given its ordinary and customary meaning to a person of ordinary skill in the art and is not to be limited to a special or customized meaning. The term specifically may refer, without limitation, to generating an event. Specifically, the event chain reaction system may be configured for producing an event in case an event chain was matched. The produced event may be published to one or more different systems. The term "publishing" as used herein is a broad term and is to be given its ordinary and customary meaning to a person of ordinary skill in the art and is not to be limited to a special or customized meaning. The term specifically may refer, without limitation, to providing an event to one or more different systems. Specifically, an event may be published to one or more different system comprised by the event chain reaction system and/or to one or more different system outside the event chain reaction system.

The term "event chain reaction" as used herein is a broad term and is to be given its ordinary and customary meaning to a person of ordinary skill in the art and is not to be limited to a special or customized meaning. The term specifically may refer, without limitation, to a process of searching certain chains in an event stream and generating a reaction when the chain is matched. The event chain reaction may have an event stream as an input. The event chain reaction may further output at least one reaction based on the input event chains. The event chain reaction may be or may comprise at least one inference rule in an expert system. The event chain reaction may be based on factual knowledge and the processing may involve forward chaining. The event chain reaction may refer to searching certain chains in an event stream and produce a reaction when the event chain is matched.

The term "event chain reaction system", as used herein, is a broad term and is to be given its ordinary and customary meaning to a person of ordinary skill in the art and is not to be limited to a special or customized meaning. The term specifically may refer, without limitation, to an arbitrary system configured for performing the event chain reaction. The event chain reaction system may specifically be configured by appropriate hardware configurations and/or by software programming. The event chain reaction system may require an event stream as input. The event chain reaction system may be a continuously running component inside an inference engine, as will be outlined in further detail below. In the event chain reaction system, it may be crucial to do chain matching based on the events received through the event stream. Thus, the chain matching may be a key element in event chain reaction system.

The event chain reaction system comprises:
- at least one communication interface configured for receiving at least one event stream, wherein the event stream comprises at least one sequence of ordered events generated by at least one analytical system, wherein each event comprises information about a change in a state of the analytical system and/or any of loaded resources;
- at least one chain reaction component comprising at least one chain matching element, wherein the chain matching element is configured for recognizing at least one chain on the event stream, wherein the chain comprises a set of ordered events to be searched, wherein a first event of the chain defines a start event, wherein the chain matching element is configured for identifying the start event in the event stream and, upon identifying the start event, the chain matching element is configured for successively determining whether the other events of the chain match to one of the events of the event stream, wherein, in case all events of the chain are matched to events of the event stream, the chain matching element is configured for triggering at least one reaction, wherein the reaction comprises generating information that a chain was matched and/or issuing a command to at least one component of the analytical system, wherein, in case one of the events of the chain is not matched, the chain matching element is configured for resetting to its initial state and waiting for the start event.

The term "communication interface" as used herein is a broad term and is to be given its ordinary and customary meaning to a person of ordinary skill in the art and is not to be limited to a special or customized meaning. The term specifically may refer, without limitation, to an arbitrary means configured for exchanging one or more item of information with at least one further component. Specifically, the communication interface may be configured, such as by appropriate hardware configurations and/or by software programming, for exchanging one or more item of information. The communication interface is specifically configured for receiving one or more item of information, such as an event stream. Further, the communication interface may be configured for exchanging the received item of information, specifically an event stream, with further components, specifically further components of the event chain reaction system, such as a chain reaction component. The communication interface may be configured for providing the events within the event stream one by one to the chain reaction component.

The term "event stream" as used herein is a broad term and is to be given its ordinary and customary meaning to a person of ordinary skill in the art and is not to be limited to a special or customized meaning. The term specifically may refer, without limitation, to a sequence of ordered events. Within the event stream, events can be present one time or multiple times. While the analytical system is running a stream of events may be created. Each new produced event may be sent to the stream. Thus, other parts of the analytical system and/or third party systems can react on it, triggering new actions that can, potentially, produce new events as the state of the analytical system or at least one of its components changes. While executing, the components of the analytical system may generate more and more events which reflect what the analytical system is doing. The longer the system runs, the more events may be "streamed". Analyzing this stream of events may provide tremendous sets of information that can be further used. An event stream may be an excellent source of data for any kind of analysis that tries to deal with both discrete value evaluation, e.g. pure data science or statistical analysis, or behavioral aspects of the data.

For example, in case of the example relating to an event indicating "rackLoaded", it may be possible to determine that a sample rack with a certain barcode was loaded at a certain point in time. It may also be possible to determine the instrument slot of the analytical system in which the sample rack was loaded and the number of samples contained by the loaded sample rack. The number of samples may be determined by counting the number of events published indicating that a sample container was identified, for example by reading the barcode of the sample container. These data may provide more information than the raw data of barcodes. For example, if this sequence may be analyzed over a longer time period, such as over one year, it may be possible to determine the time at which customers load sample racks. It may be possible to determine the time at which specific samples were loaded to perform a specific test, such as sample for HIV-tests. It may be possible to determine the maximum number of test which are processed in a certain time span, such as during an 8-hour shift.

The events comprised by the event stream may be ordered with respect to their time stamp. Within the event stream, the events may be present multiple times. In case the sequences of events of the event chain may be found or matched within the event stream, a reaction may be produced by the event chain reaction system.

The event stream may be a fixed event stream. The fixed event stream may comprise a finite number of events, wherein the number of events may be constant. The fixed event stream may comprise events batched together from a source, such as from a log file, a problem report or the like. The fixed event stream may be used by system which are not constantly connected to the event chain reaction system, specifically to the communication interface. Thus, the fixed even steam may be used by components which are only occasionally connected to a cloud-based infrastructure, as will be outlined in further detail below. The components may batch the events and send them from time to time.

The event stream may be an unbounded event stream. The unbounded event stream may comprise an infinite number of events. For example, the unbounded event stream may comprise events which are continuously appended to the event stream. The unbounded event stream may be used by data streaming system for real time analysis. Thus, unbounded event stream may be produced by connected instruments which are constantly streaming events.

As outlined above, the event stream comprises the at least one sequence of ordered events generated by at least one analytical system. The term "analytical system" as used herein is a broad term and is to be given its ordinary and customary meaning to a person of ordinary skill in the art and is not to be limited to a special or customized meaning. The term specifically may refer, without limitation, to a system comprising one or more components configured for performing at least one analytical function. Specifically, the analytical function may be or may comprise one or more of handling loaded resources, such as a sample contained in a sample container, processing loaded resources or samples, determining a presence and/or a concentration of an analyte in a sample. In particular, the analytical system may comprise a plurality of components which may be configured for interacting together to perform the at least one analytical function. At least one of the components of the analytical system may be configured for performing at least one of the analytical functions and thus, the analytical system may be configured for performing a plurality of different analytical functions. The analytical system may comprise at least one sensor configured for detecting a change of state of the analytical system or any parts thereof. For example, one or more of the components of the analytical system may be or may comprise at least one sensor configured for sensing the state of the analytical system and/or any parts thereof, and further for producing a signal indicating the change of state. The sensor may be at least one of: an optical sensor, such as an optical reading device configured for reading optical identifiers, such as barcodes or the like; an electronic sensor; a temperature sensor. Thus, the analytical system may be, specifically according to the target architecture for analytical systems, capable of streaming data in the shape of events. Further, the analytical system may comprise one or more of an analyzer, a pre-post processor or a point-of-care device.

The even chain reaction system comprises at least one chain reaction component comprising at least one chain matching element. The term "chain reaction component" as used herein is a broad term and is to be given its ordinary and customary meaning to a person of ordinary skill in the art and is not to be limited to a special or customized meaning. The term specifically may refer, without limitation, to a computing device configured, such as by hardware configuration and/or software programming, for performing the event chain reaction. Events within the event stream may be sent, one by one, to the chain reaction component. The chain reaction component may know all the chains that the system must recognize on an event stream. The chain reaction component may be configured for storing at least one item of information. Specifically, the chain reaction component may be configured for storing the event chain to be searched. The chain reaction component may be configured for storing all the event chains that the even chain reaction system must recognize on the event stream.

As outlined above, out of the information of the event stream it may be necessary to detect or match certain chains, such as patterns, inside the event stream. Specifically, this may be useful when the intention is to identify chains that indicate that some properties of the system are changing such as that the performance is degrading or that some hardware component is not performing as expected anymore or the like.

The term "chain matching element" as used herein is a broad term and is to be given its ordinary and customary meaning to a person of ordinary skill in the art and is not to be limited to a special or customized meaning. The term specifically may refer, without limitation, to an arbitrary device configured for event chain matching. Specifically, event chain matching may refer to a process where a specific event chain is searched within the event stream. The chain matching component may be configured for looking at the event stream searching for the specific event chain and/or event pattern. The chain matching element may be configured for matching an event chain. The chain matching element may produce a match, which may then produce the reaction in a downstream system. The event chain reaction system may continuously evaluate any received event stream or new event of a known stream for chain matching.

The chain comprises a set of ordered events to be searched. Each chain may comprise at least one starter, at least one event sequence and at least one ender. The first event of the chain defines a start event.. The term "start event" or "first event", as used herein is a broad term and is to be given its ordinary and customary meaning to a person of ordinary skill in the art and is not to be limited to a special or customized meaning. The term specifically may refer, without limitation, to a specific event of the event chain to be searched, which is ordered in the first place of the event chain. Thus, the start event may define the beginning of the event chain to be searched. The chain matching element is configured for identifying the start event in the event stream. The term "identifying the start event" as used herein is a broad term and is to be given its ordinary and customary meaning to a person of ordinary skill in the art and is not to be limited to a special or customized meaning. The term specifically may refer, without limitation, to comparing at least one property of the received event or events with at least one property of the first event in the chain. The property of the first event may be a type of the event. In addition for identification, the chain matching element may check if at least one attribute of the received event or events matches at least one attribute of the first event. Preferably, the chain matching element checks if all attributes of the received event or events match the attributes of the first events. The term "attribute" as used herein is a broad term and is to be given its ordinary and customary meaning to a person of ordinary skill in the art and is not to be limited to a special or customized meaning. The term specifically may refer, without limitation, to payload data. For example, the attribute may comprise one or more of a time stamp, an originator identifier and/or a barcode of a sample container or a sample rack. The attribute may also refer to a slot or a position number of loaded resources. The chain matching element may wait until an event of the specified type and/or with the specified attributes in the payload is received. Upon identifying the start event, the chain matching element is configured for successively determining whether the other events of the chain match to one of the events of the event stream. In case no start event may be matched, this instance of the chain matching element may ignore all the received events.

Further, after receiving the start event, a chain may be composed of a sequence of events. During the matching process, the chain matching element may check that the received event match or events matches the next event in the event chain. The term "check if the received event matches the next event" as used herein is a broad term and is to be given its ordinary and customary meaning to a person of ordinary skill in the art and is not to be limited to a special or customized meaning. The term specifically may refer, without limitation, to comparing at least one property of the received event or events with at least one property of the event in the chain. The property of the event may be a specific type of event. The chain matching element may also check that the attributes of the received events matches the attributes of the next event in the event chain.

The chain matching element may be configured for detect lineal and/or non-lineal sequences. Non-lineal sequences may be sequences wherein between two events of the event stream matched to two successive events of the chain there may be multiple other events, in particular events associated with a first event of said two events of the event stream. The other event may be associated to said first event with respect to the type of the events. For example, if the first event may be of the type "rack loaded", the following events may indicate that one or more sample container in the loaded rack were identified, and, thus, these events may be associated to the first event of type "rack loaded".

The at least one ender may identify the last event on the event chain. If this event may be matched, specifically together with its attributes, the event chain may be considered as matched and the reaction may be triggered. The chain matching element may be configured for identifying the last event on the event stream. The term "identifying the last event" as used herein is a broad term and is to be given its ordinary and customary meaning to a person of ordinary skill in the art and is not to be limited to a special or customized meaning. The term specifically may refer, without limitation, to comparing at least one property of the received event or events with at least one property of the last event in the chain. The property of the last event may be a type of the event. In addition for identification, the chain matching element may check if at least one attribute of the received event or events matches at least one attribute of the last event. Preferably, the chain matching element checks if all attributes of the received event or events match the attributes of the last event. In case the chain matching element may identify the start event, the sequence of events and the last event on the event stream, the event chain is matched. The chain matching element may produce a match indicating that the event chain was matched.

The chain reaction component comprises a plurality of chain matching elements. Each chain matching element may be configured for matching an event chain. Each chain matching elements may produce a match, which may then produce the reaction in a downstream system, as will be outlined in further detail below. At this point, the responsibility of the event chain reaction system may be finished.

For each received event, the chain reaction component may forward the event to the different chain matching elements in parallel. The event chain reaction system may be able to perform multiple event chain matchings at the same time, potentially provoking multiple reactions at the same time.

The chain matching element may be stateful from the moment that the start event is received. The chain matching element may be configured for remembering what the next expected event is. At the moment that one received event is not matching anymore the next expected event, then the event chain may be considered broken and the chain matching element may return to its initial state and may start waiting again for the start event.

The chain reaction component may comprise a plurality of chain matching elements, wherein the chain reaction component is configured for multiple chain matching in parallel. Consequently, the chain reaction component may be configured for triggering multiple reactions at the same time.

The event chain reaction system may be an inference engine in an expert system. The event chain reaction may be an inference rule used by the inference engine. As used herein, the term "expert system" is a broad term and is to be given its ordinary and customary meaning to a person of ordinary skill in the art and is not to be limited to a special or customized meaning. The term specifically may refer, without limitation, to a computer or computer system configured for solving complex problems using artificial intelligence. The expert systems may be a computer applications developed to solve complex problems in a particular domain, at the level of extra-ordinary human intelligence and expertise. The characteristics of the expert systems may comprise a high performance, an understandable, reliable and highly responsive application.

The expert systems may be capable of one or more of advising, instructing and assisting human in decision making, demonstrating, deriving a solution, diagnosing, explaining, interpreting input, predicting results, justifying the conclusion or suggesting alternative options to a problem.

The expert system may comprise at least one knowledge base, at least one inference engine and at least one user interface.

The term "knowledge base", as used herein, is a broad term and is to be given its ordinary and customary meaning to a person of ordinary skill in the art and is not to be limited to a special or customized meaning. The term specifically may refer, without limitation, to technology configured for storing knowledge such as complex structured and unstructured information. The at least one knowledge base may comprise domain-specific and high-quality knowledge. The knowledge may be required to exhibit intelligence. The knowledge of the expert system may comprise a collection of highly accurate and precise knowledge. Specifically, the knowledge may refer to data as a collection of facts. The information may be organized as data and/or facts about the task domain. The term "knowledge", as used herein, is a broad term and is to be given its ordinary and customary meaning to a person of ordinary skill in the art and is not to be limited to a special or customized meaning. The term specifically may refer, without limitation, to a combination of data and information, that has been acquired through past experience.

The at least one knowledge base of the expert system may be a store of both, factual and heuristic knowledge. As used herein, the term "factual knowledge" is a broad term and is to be given its ordinary and customary meaning to a person of ordinary skill in the art and is not to be limited to a special or customized meaning. The term specifically may refer, without limitation, to information widely accepted by knowledge engineers and scholars in the task domain. As further used herein, the term "heuristic knowledge" is a broad term and is to be given its ordinary and customary meaning to a person of ordinary skill in the art and is not to be limited to a special or customized meaning. The term specifically may refer, without limitation, to one or more of a practice, an accurate judgement and an ability of evaluation and/or guessing.

The knowledge may be represented by organizing and/or formalizing the knowledge in the knowledge base. For example, the knowledge may be represented in the form of if-then-else rules.

The knowledge of the expert system may be acquired in a high-quality, complete and accurate information stored in the knowledge base. The knowledge base may be formed by readings from various experts, scholars, and the knowledge engineers. The knowledge engineer may refer to a person with the qualities of deep understanding, quick learning, and case analyzing skills. The knowledge engineer may acquire information from subject expert by recording, interviewing, and observing him at work, etc. The knowledge engineer may then categorize and organize the information in a meaningful way, for example in the form of if-then-else rules, to be used by the inference machine.

The term "inference engine", as used herein, is a broad term and is to be given its ordinary and customary meaning to a person of ordinary skill in the art and is not to be limited to a special or customized meaning. The term specifically may refer, without limitation, to a component of the expert system configured for applying inference rules to the knowledge base in order to determine new information. The term "inference rule", as used herein, is a broad term and is to be given its ordinary and customary meaning to a person of ordinary skill in the art and is not to be limited to a special or customized meaning. The term specifically may refer, without limitation, to at least one logical rule which typically may be represented as IF-THEN rule. The at least one inference engine may use efficient procedures and inference rules for deducting a correct and/or flawless solution. Specifically, in case of a knowledge-based expert system, the inference engine may acquire and manipulate the knowledge from the knowledge base to arrive at a particular solution. In case of a rule based expert system, the at least one inference engine may apply inference rules repeatedly to the facts, which may obtained from earlier inference rule application. The inference engine may further add new knowledge into the knowledge base if required. The inference engine may also resolve inference rules conflict when multiple inference rules are applicable to a particular case. Preferably, the interference engine is both knowledge and rule based. The event chain reaction may be a continuously running component inside the inference engine.

In order to recommend a solution, the at least one inference engine may use forward chaining. The event chain reaction may be based on factual events knowledge and may be forward chaining. The forward chaining may refer to a strategy of the expert system to answer the question of what happens next. The inference engine may follow a chain of conditions and/or derivations and may finally deduce the outcome. The inference engine may consider all the facts and inference rules, and may sort them before concluding to a solution. The strategy of forward chaining may be followed for working on conclusions, results and/or effects. For example, a prediction of share market status may be an effect of changes in interest rates.

The term "user interface", as used herein, is a broad term and is to be given its ordinary and customary meaning to a person of ordinary skill in the art and is not to be limited to a special or customized meaning. The term specifically may refer, without limitation, to at least one interface configured for providing interaction between user of the expert system and the expert system itself. The user interface may generally be a natural language processing to be used by the user who may be well-versed in the task domain. The user of the expert system may not necessarily be an expert in artificial intelligence. The user interface may explain how the expert system has arrived at a particular recommendation. The explanation may appear in one of the following forms: natural language displayed on screen; verbal narrations in natural language; listing of rule numbers displayed on the screen. The user interface may enable tracing the credibility of the deductions.

The user interface of the expert system may help the users to accomplish their goals in shortest possible way. The user interface may be designed to work for user's existing or desired work practices. The technology of the user interface may be adaptable to user's requirements. The user interface may enable efficient use of user input.

In a further aspect of the present invention, a system for monitoring and/or controlling is disclosed. The system is configured for monitoring and/or controlling at least one feature of at least one component of an analytical system.

As outlined above, the feature may be related to the function of the component of the analytical system. For example, the feature may describe a performance of the component of the analytical system. Alternatively or additionally, the feature may also indicate a correct functioning of the component of the analytical system. The feature of the at least one component of the analytical system may specifically be detected by at least one event chain reaction system. Further, the chain of specific events may be created to detect the feature, so to be able to detect the issue of the at least one component of the analytical system. The issue of the component of the analytical system may be at the hardware or the software level or may be a combination of both.

The feature of the at least one component of the analytical system may be qualitatively and/or quantitatively determined by the system for monitoring and/or controlling. The term "monitoring and/or controlling" as used herein is a broad term and is to be given its ordinary and customary meaning to a person of ordinary skill in the art and is not to be limited to a special or customized meaning. The term specifically may refer, without limitation, to a process of retrieving an item of information and determining at least one recommended action based on the retrieved item of information. Specifically, the system for monitoring and/or controlling may be configured for retrieving at least one event stream from the analytical system, analyzing the at least one event stream and, further, for providing the at least one recommended action based on the retrieved event stream to the analytical system. The analyzing of the event stream may refer to one or more of a statistical analysis of discrete values of the event stream and/or a statistical analysis of behavioral aspects of the event stream.

The system for monitoring and/or controlling comprises at least one cloud-based infrastructure. The term "cloud-based infrastructure" as used herein is a broad term and is to be given its ordinary and customary meaning to a person of ordinary skill in the art and is not to be limited to a special or customized meaning. The term specifically may refer, without limitation, to a computing infrastructure which is accessible for the analytical system via at least one network. Specifically, the cloud-based infrastructure may provide a computing infrastructure which is separated from the analytical system but which may be configured for exchanging data between the cloud-based infrastructure and the analytical system via the at least one network. The cloud-based infrastructure may refer to a common infrastructure deployed in a cloud which serves as the foundation to build other applications or services on top of it.

The cloud-based infrastructure comprises at least one first communication interface configured for receiving a plurality of events.

The cloud-based infrastructure further comprises at least one storage unit configured for storing the received events. The term "storage unit" as used herein is a broad term and is to be given its ordinary and customary meaning to a person of ordinary skill in the art and is not to be limited to a special or customized meaning. The term specifically may refer, without limitation, to an arbitrary device configured for retaining data. Specifically, the storage unit may be configured for retaining the at least one event stream. The cloud-based infrastructure may be configured for extracting and transforming the events of the event stream to a common and/or homogenous data structure. The storage unit may be configured for extracting the events. The storage unit may be configured for transforming the events into a generic structure. The storage unit may be configured for storing the events in the event store. The storage unit may be configured for storing each event of the at least one event stream, specifically each event may be stored wrapped into a data format which provides metadata about it, such as origin, version, reception time, sequence identification and the like. The cloud-based infrastructure may be configured for storing the events so that historical analysis can be performed later. For example, storing time may be one year of data for each instrument. This may allow future machine learning models to already have access to historical data. The events may be stored in a so-called event store.

The cloud-based infrastructure comprises at least one event chain reaction system according to the present invention, such as according to any one of the embodiments disclosed above and/or any one of the embodiments disclosed in further detail below. Thus, for definitions and embodiments of the system reference is made to definitions and embodiments of the event chain reaction system.

The cloud-based infrastructure comprises at least one streaming platform configured for streaming events to the event chain reaction system, wherein the event chain reaction system is configured for detecting the feature of the component of the analytical system by matching the chain to the event stream. The term "streaming platform" as used herein is a broad term and is to be given its ordinary and customary meaning to a person of ordinary skill in the art and is not to be limited to a special or customized meaning. The term specifically may refer, without limitation, to any means configured for providing subscription-based services. Specifically, the streaming platform is configured, such as by hardware configuration and/or by software programming, for streaming events to the event chain reaction system. The streaming platform may further be configured for streaming the events of the event stream to any other part of the system for monitoring and/or controlling which subscribed to the streaming platform. The streaming platform may be configured for streaming events to different consumers which might subscribe to specific ones or to all events in the event stream. Consumers may be instrument-specific or general purpose. Specifically, as outlined above, the chain reaction component may comprise one or more chain matching elements, wherein each of the chain reaction components is configured for chain matching. The chain matching elements are configured for parallel processing. The chain matching elements may be the main subscribers of the event.

The streaming platform may be configured for streaming events to a plurality of subscribers in parallel. The cloud-based infrastructure may further comprise at least one extraction and storage unit configured for transforming the events into a generic data structure. The cloud-based infrastructure may also comprise at least one notification bus configured for exchange data between components of the cloud-based infrastructure.

The cloud-based infrastructure may be configured for one or more of: receiving the event streams from all the analytical systems in the field, specifically from all analytical system located worldwide; extracting and transforming the events of the event stream to a common and/or homogenous data structure; storing the events of the even stream, specifically data for each instrument, such that historical analysis may be performed retrospectively, specifically retrospectively for up to one year; streaming the events, specifically the event stream, to different consumers which might subscribe to specific events and/or to all of the events in the event stream; providing an infrastructure necessary to allow applications to exchange events of the event stream between the different applications, for example events generated out of the processing of the event stream or triggered by other operations.

The system for monitoring and/or controlling comprises at least one downstream system. The term "downstream" as used herein is a broad term and is to be given its ordinary and customary meaning to a person of ordinary skill in the art and is not to be limited to a special or customized meaning. The term specifically may refer, without limitation, to a location outside of the cloud. After the processing, in particular the parallel processing, some operations can be triggered on downstream processing applications. The term "downstream system" as used herein is a broad term and is to be given its ordinary and customary meaning to a person of ordinary skill in the art and is not to be limited to a special or customized meaning. The term specifically may refer, without limitation, to at least one device and/or application configured for processing and/or reaction on the processing of the events outside the cloud. The downstream system may be or may be based on an arbitrary decision logic. Specifically, the downstream system is configured, such as by hardware configuration and/or by software programing, for performing at least one action based on the reaction. The downstream system may be configured for receiving, such as via at least one interface, specifically via at least one second interface, the at least one reaction triggered by the event chain reaction system. The downstream system may be configured for determining and/or deriving the at least one action from the reaction. The downstream system may be configured for relating the reaction from the event chain reaction system to the at least one action, for example by using one or more of a decision tree, a decision table or a decision rule, such as an if-then-else rule. Downstream processing may refer to a reaction on the processing of the events. For example, if a known event pattern is detected, a notification may be sent to a global customer support operator such that the support operator may take the necessary actions.

The cloud-based infrastructure further comprises at least one second interface which is configured for providing the at least one reaction triggered by the event chain reaction system to the downstream system. The downstream system is configured for performing at least one action based on the reaction, wherein the action is one or more of generating a notification about the feature to at least one operator and/or controlling the feature of the component of the analytical system by adapting at least one property of the component of the analytical system.

The system may further comprise at least one prediction unit configured for predicting future behavior of the analytical system based on event data. The prediction unit may be configured for recognizing patterns and/or regularities in the event data, such as in the matched event chains. The prediction unit may be configured for predicting the future behavior, for example the future events in the event stream, based on the recognized patterns and/or regularities in the event data.

The system for monitoring and/or controlling may be embodied as an event stream processing suite (ESPS) which may be configured for processing the at least one event stream and, further, for providing extended functionality and/or services to different areas. ESPS may be implemented as an expert system which may refer to an artificial intelligence solution. The event chain reaction system may be an inference engine in the expert system and the event chain reaction may be an inference rule used by the inference engine. The ESPS may be an expert system with rule-based approach for the factual knowledge.

The ESPS may further comprise the cloud-based infrastructure and further applications and/or services. For example, applications may use the cloud-based infrastructure and may provide specific functionality, such as one or more of subscribing to specific events from the event stream, specifically subscribing to an even "Run Batch Started" and/or to an event "Run Batch Finished" to detect degradation in the duration of a batch run, publishing events that might be of interest to other applications, specifically publishing an event "Run Batch Degradation Detected", performing application-specific functionality, specifically entering a new chain and/or an event pattern which may produce a known error in the system.

The system for monitoring and/or controlling may further comprise at least one notification bus. The notification bus may be configured for enabling system level communication between the system's components, specifically for exchanging system level notifications, such as notifications about a received package, a new created pattern, an added comment or the like. It may be also possible to use the at least one streaming platform for system level communication between the system's components, specifically for exchanging system level notifications. Thus, in this case, the at least one streaming platform may be used for exchanging both types of events, system- and/or instrument-produced events.

The system for monitoring and/or controlling may be used for one or more of monitoring and/or controlling of at least one medical system such as a diagnosis system configured for deducing cause of disease from observed data or a medical system for conducting medical operations on humans; monitoring and/or controlling of at least one monitoring system such as a monitoring system configured for comparing data continuously with observed system or prescribed behavior such as leakage monitoring in pipelines; monitoring and/or controlling of at least process control system configured for controlling a physical process based on monitoring; monitoring and/or controlling of at least one analysis system configured for determining faults in vehicles and/or computers; monitoring and/or controlling of at least one finance or commerce system configured for one or more of detecting of fraud, suspicious transactions, stock market trading, airline scheduling, cargo scheduling; monitoring and/or controlling of at least one design system configured for one or more of designing a camera lens design or automobile design.

In a further aspect of the invention, a computer implemented method for determining at least one feature of at least one component of an analytical system is disclosed. In the method, at least one event chain reaction system according to the present invention, such as according to any one of the embodiments disclosed above and/or any one of the embodiments disclosed in further detail below, is used. Thus, for definitions and embodiments of the method reference is made to definitions and embodiments of the event chain reaction system.

The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

The method comprises the following steps:
i) providing at least one event stream via the communication interface, wherein the event stream comprises at least one sequence of ordered events generated by the component of the analytical system, wherein each event comprises information about a change in a state of the analytical system and/or any of the loaded resources;
ii) determining the feature of the component of the analytical system by recognizing at least one chain on the event stream by using the chain matching element of the chain reaction component, wherein the chain comprises a set of ordered events to be searched, wherein a first event of the chain defines a start event, wherein the recognizing comprises identifying the start event in the event stream and, upon identifying the start event, successively determining whether the other events of the chain match to one of the events of the event stream;
iii) triggering at least one reaction, in case all events of the chain are matched to events of the event stream, wherein the reaction comprises generating information that a chain was matched and/or issuing a command to at least one component of the analytical system, or resetting of the chain matching element to its initial state and waiting for the start event, in case one of the events of the chain is not matched.

As outlined above, the method comprises determining the feature of the component of the analytical system by recognizing the at least one chain on the even stream. The chain may specifically be an ordered set of events indicative for a feature of the analytical system.

In a further aspect of the present invention, a computer program for determining at least one feature of at least one component of an analytical system is disclosed. The computer program is configured for causing a computer or computer network to fully or partially perform the method for determining at least one feature of at least one component of an analytical system according to the present invention, such as according to any one of the embodiments disclosed above and/or any one of the embodiments disclosed in further detail below, when executed on the computer or computer network. The computer program is further configured to perform at least steps i) to iii) of the method for determining at least one feature of at least one component of an analytical system according to the present invention, such as according to any one of the embodiments disclosed above and/or any one of the embodiments disclosed in further detail below.

In a further aspect of the present invention, a computer implemented method for monitoring and/or controlling at least one feature of at least one component of an analytical system is disclosed. In the method, at least one system for monitoring and/or controlling according to the present invention, such as according to any one of the embodiments disclosed above and/or any one of the embodiments disclosed in further detail below.

The method comprises the following steps which, as an example, may be performed in the given order. It shall be noted, however, that a different order is also possible. Further, it is also possible to perform one or more of the method steps once or repeatedly. Further, it is possible to perform two or more of the method steps simultaneously or in a timely overlapping fashion. The method may comprise further method steps which are not listed.

The method comprises the following steps:
I) receiving a plurality of events via the first communication interface of the cloud-based infrastructure;
II) storing the received events with the storage unit of the cloud-based infrastructure;
III) streaming events to the event chain reaction system via the streaming platform;
IV) detecting the feature of the component of the analytical system by matching the chain to the event stream with the event chain reaction system;
V) providing the at least one reaction triggered by the event chain reaction system to the downstream system via the second interface;
VI) the downstream system performing at least one action based on the reaction, wherein the action is one or more of generating a notification about the feature to at least one operator and/or controlling the feature of the component of the analytical system by adapting at least one property of the component of the analytical system.

The method may further comprise transforming the events into a generic data structure by using at least one extraction and storage unit of the cloud-based infrastructure. Further, the method may comprise an exchange of data between components of the cloud-based infrastructure via at least one notification bus.

The method may further comprise, specifically in steps I), II) and/or III), extracting and/or storing the received events. Thus, received events may be extracted, such as in case of fixed streams, transformed into a generic structure and stored in an event store, such as within the at least one storage unit. The original event may be kept wrapped into a data format which may provide metadata about the event, such as origin, version, reception time and/or sequence identification.

The method may further comprise using the at least one streaming platform, specifically in step III). Once the event has been extracted, transformed and stored, the event may be sent to the streaming platform which may ensure that the event is streamed to all the subscribers which are interested on it. Thus, the at least one streaming platform may serve as the inference engine in the expert system

The method may further comprise a parallel processing, specifically a parallel processing in steps IV). Each subscriber may receive a copy of the original event and may process the event in parallel to the other subscriber. For example, the event chain reaction system may be one of the subscriber of the event. The event chain reaction system may be responsible for the chain matching.

The method may further comprise a downstream processing, specifically in step V) and VI). After the parallel processing of the events may be performed, some operations may be triggered on downstream processing applications, which may potentially be deployed outside the cloud-based infrastructure. The downstream processing may be meant to react on the processing of the events. For example, if a known event pattern is detected, a notification may be sent to a global customer support operator such that the support operator may take the necessary actions.

In a further aspect of the present invention, a computer program for monitoring and/or controlling at least one feature of at least one component of an analytical system is disclosed. The computer program is configured for causing a computer or computer network to fully or partially perform the method for monitoring and/or controlling at least one feature of at least one component of an analytical system according to the present invention, such as according to any one of the embodiments disclosed above and/or any one of the embodiments disclosed in further detail below, when executed on the computer or computer network. The computer program is configured to perform at least steps I) to VI) of the method for monitoring and/or controlling at least one feature of at least one component of an analytical system according to the present invention, such as according to any one of the embodiments disclosed above and/or any one of the embodiments disclosed in further detail below.

The methods and devices according to the present invention provide a large number of advantages over similar methods and devices known in the art. Specifically, the methods and devices may provide a solution which may be more flexible in terms of detecting issues and/or trends even with a mutated sequence of events. The system for monitoring and/or controlling may be or may comprise an expert systems based on factual knowledge bases which may allow combinations of different if-then-else based rules to cover different scenarios of the same error. Extending this set of rules and including a prediction unit which allows experts to verify the results of the prediction may enable the system to move from a reactive to a predictive solution.

Further, the methods and device according to the present invention may implement an event-driven architecture which may allow propagating instrument information to other software components in a way that is natural to the system. Specifically, event-driven architectures may describe system functionalities as the result of exchanging a set of events which reflects changes on the state of the different system components. Each event may potentially trigger at least one reaction on one or more components of the analytical system which subscribes to the system. Further, event-driven architectures may focus on the behavioral aspects of the system rather than on the discrete values generated by the different components of the analytical system. Specifically, events may be written in past sense to express the fact that something happened or something changed. Thus, events may not only describe a fact that happened, but may also provide insights on what happened in their payload. While the analytical system is running, a stream of events may be created. Each new produced event may be sent to the event stream, such that other parts of the system and/or even third-party systems may be able to react on the event, triggering new actions that may potentially produce new events as the state of the analytical system and/or some its component changes. The event stream of events may be an excellent source of data for any kind of analysis that tries to deal with both discrete value evaluation, such as pure data science and/or statistical analysis, and/or behavioral aspects of the data.

In general, analytical systems may be composed of both software and hardware components and may be able of producing events that represents changes in the state of the analytical system and/or any components thereof. Hardware may be, by nature, event-driven, such that a sensor may produce signals based on physical interactions. Signals may be just one type of event, but there may be many more.

Analyzing the event stream may provide tremendous sets of information that may further be used. This information may provide more information than just the raw data of barcodes or the like. If the sequence of events may be analyzed over a period of time, such as over at least one year, common patterns may be identified and further information may be retrieved. For example, an information may be retrieved identifying at which time customers usually load racks to the analytical system. The information may further comprise information about what kind of test are performed on samples loaded at a specific point in time. The information may also refer to a maximum number of performed test during a specific time period, for example within an eight hours shift. The information may be used to better configure the system to match customer usage and improve usability aspects of the system. The performance of the analytical system may be improved by predicting what the user is going to do next. By using the methods and devices according to the present invention, it may be possible to build a personalized predictive maintenance.

Further disclosed and proposed herein is a computer program including computer-executable instructions for performing the method according to the present invention in one or more of the embodiments enclosed herein when the program is executed on a computer or computer network. Specifically, the computer program may be stored on a computer-readable data carrier and/or on a computer-readable storage medium.

As used herein, the terms "computer-readable data carrier" and "computer-readable storage medium" specifically may refer to non-transitory data storage means, such as a hardware storage medium having stored thereon computer-executable instructions. The computer-readable data carrier or storage medium specifically may be or may comprise a storage medium such as a random-access memory (RAM) and/or a read-only memory (ROM).

Thus, specifically, one, more than one or even all of method steps a) to d) as indicated above may be performed by using a computer or a computer network, preferably by using a computer program.

Further disclosed and proposed herein is a computer program product having program code means, in order to perform the method according to the present invention in one or more of the embodiments enclosed herein when the program is executed on a computer or computer network. Specifically, the program code means may be stored on a computer-readable data carrier and/or on a computer-readable storage medium.

Further disclosed and proposed herein is a data carrier having a data structure stored thereon, which, after loading into a computer or computer network, such as into a working memory or main memory of the computer or computer network, may execute the method according to one or more of the embodiments disclosed herein.

Further disclosed and proposed herein is a computer program product with program code means stored on a machine-readable carrier, in order to perform the method according to one or more of the embodiments disclosed herein, when the program is executed on a computer or computer network. As used herein, a computer program product refers to the program as a tradable product. The product may generally exist in an arbitrary format, such as in a paper format, or on a computer-readable data carrier and/or on a computer-readable storage medium. Specifically, the computer program product may be distributed over a data network.

Finally, disclosed and proposed herein is a modulated data signal which contains instructions readable by a computer system or computer network, for performing the method according to one or more of the embodiments disclosed herein.

Referring to the computer-implemented aspects of the invention, one or more of the method steps or even all of the method steps of the method according to one or more of the embodiments disclosed herein may be performed by using a computer or computer network. Thus, generally, any of the method steps including provision and/or manipulation of data may be performed by using a computer or computer network. Generally, these method steps may include any of the method steps, typically except for method steps requiring manual work, such as providing the samples and/or certain aspects of performing the actual measurements.

Specifically, further disclosed herein are:
- a computer or computer network comprising at least one processor, wherein the processor is adapted to perform the method according to one of the embodiments described in this description,
- a computer loadable data structure that is adapted to perform the method according to one of the embodiments described in this description while the data structure is being executed on a computer,
- a computer program, wherein the computer program is adapted to perform the method according to one of the embodiments described in this description while the program is being executed on a computer,
- a computer program comprising program means for performing the method according to one of the embodiments described in this description while the computer program is being executed on a computer or on a computer network,
- a computer program comprising program means according to the preceding embodiment, wherein the program means are stored on a storage medium readable to a computer,
- a storage medium, wherein a data structure is stored on the storage medium and wherein the data structure is adapted to perform the method according to one of the embodiments described in this description after having been loaded into a main and/or working storage of a computer or of a computer network, and
- a computer program product having program code means, wherein the program code means can be stored or are stored on a storage medium, for performing the method according to one of the embodiments described in this description, if the program code means are executed on a computer or on a computer network.

Summarizing and without excluding further possible embodiments, the following embodiments may be envisaged:
Embodiment 1: An event chain reaction system comprising
   - at least one communication interface configured for receiving at least one event stream, wherein the event stream comprises at least one sequence of ordered events generated by at least one analytical system, wherein each event comprises information about a change in a state of the analytical system and/or any of loaded resources;
   - at least one chain reaction component comprising at least one chain matching element, wherein the chain matching element is configured for recognizing at least one chain on the event stream, wherein the chain comprises a set of ordered events to be searched, wherein a first event of the chain defines a start event, wherein the chain matching element is configured for identifying the start event in the event stream and, upon identifying the start event, the chain matching element is configured for successively determining whether the other events of the chain match to one of the events of the event stream, wherein, in case all events of the chain are matched to events of the event stream, the chain matching element is configured for triggering at least one reaction, wherein the reaction comprises generating information that a chain was matched and/or issuing a command to at least one component of the analytical system, wherein, in case one of the events of the chain is not matched, the chain matching element is configured for resetting to its initial state and waiting for the start event.
Embodiment 2: The event chain reaction system according to the preceding embodiment, wherein the chain reaction component comprises a plurality of chain matching elements, wherein the chain reaction component is configured for multiple chain matching in parallel.
Embodiment 3: The event chain reaction system according to the preceding embodiment, wherein the chain reaction component is configured for triggering multiple reactions at the same time.
Embodiment 4: The event chain reaction system according to any one of the preceding embodiments, wherein each of the event comprises a time stamp when the event was produced.
Embodiment 5: The event chain reaction system according to any one of the preceding embodiments, wherein each of the event comprises an originator identifier indicating which identity has produced the event.
Embodiment 6: The event chain reaction system according to any one of the preceding embodiments, wherein the chain is an ordered set of events indicative for a feature of the analytical system.
Embodiment 7: The event chain reaction system according to any one of the preceding embodiments, wherein the information about a change in a state of the analytical system is at least one of an operational information or a sensor signal generated by at least one sensor of the analytical system.
Embodiment 8: The event chain reaction system according to the preceding embodiment, wherein the information about a change in a state of the analytical system is at least one information selected from the group consisting of: a sensor signal indicating that performance is changing; a sensor signal indicating that a sample rack is loaded; a sensor signal indicating that sample container is identified, a sensor signal indicating that over temperature is detected, a sensor signal indicating that a fuse is broken, a sensor signal indicating that performance is changing, a sensor signal indicating that at least one hardware component of the analytical system performs differently as expected.
Embodiment 9: A system for monitoring and/or controlling at least one feature of at least one component of an analytical system, wherein the system comprises at least one cloud-based infrastructure, wherein the cloud-based infrastructure comprises at least one first communication interface configured for receiving a plurality of events, wherein the cloud-based infrastructure comprises at least one storage unit configured for storing the received events, , wherein the cloud-based infrastructure comprises at least one event chain reaction system according to any one of the preceding embodiments, wherein the cloud-based infrastructure comprises at least one streaming platform configured for streaming events to the event chain reaction system, wherein the event chain reaction system is configured for detecting the feature of the component of the analytical system by matching the chain to the event stream, wherein the system comprises at least one downstream system, wherein the cloud-based infrastructure comprises at least one second interface which is configured for providing the at least one reaction triggered by the event chain reaction system to the downstream system, wherein the downstream system is configured for performing at least one action based on the reaction, wherein the action is one or more of generating a notification about the feature to at least one operator and/or controlling the feature of the component of the analytical system by adapting at least one property of the component of the analytical system.
Embodiment 10: The system for monitoring and/or controlling according to the preceding embodiment, wherein the streaming platform is configured for streaming events to a plurality of subscribers in parallel.
Embodiment 11: The system for monitoring and/or controlling according to any one of two the preceding embodiments, wherein the cloud-based infrastructure comprises at least one extraction and storage unit configured for transforming the events into a generic data structure.
Embodiment 12: The system for monitoring and/or controlling according to any one of the three preceding embodiments, wherein the cloud-based infrastructure comprises at least one notification bus configured for exchange data between components of the cloud-based infrastructure.
Embodiment 13: The system for monitoring and/or controlling according to any one of the four preceding embodiments, wherein the system comprises at least one prediction unit configured for predicting future behavior of the analytical system based on event data.
Embodiment 14: A computer implemented method for determining at least one feature of at least one component of an analytical system, wherein in the method at least one event chain reaction system according to any one of the preceding embodiments referring to an event chain reaction system is used, wherein the method comprises the following steps:
   i) providing at least one event stream via the communication interface, wherein the event stream comprises at least one sequence of ordered events generated by the component of the analytical system, wherein each event comprises information about a change in a state of the analytical system and/or any of the loaded resources;
   ii) determining the feature of the component of the analytical system by recognizing at least one chain on the event stream by using the chain matching element of the chain reaction component, wherein the chain comprises a set of ordered events to be searched, wherein a first event of the chain defines a start event, wherein the recognizing comprises identifying the start event in the event stream and, upon identifying the start event, successively determining whether the other events of the chain match to one of the events of the event stream;
   iii) triggering at least one reaction, in case all events of the chain are matched to events of the event stream, wherein the reaction comprises generating information that a chain was matched and/or issuing a command to at least one component of the analytical system, or resetting of the chain matching element to its initial state and waiting for the start event, in case one of the events of the chain is not matched.
Embodiment 15: The method according to the preceding embodiment, wherein the chain is an ordered set of events indicative for a feature of the analytical system.
Embodiment 16: Computer program for determining at least one feature of at least one component of an analytical system, configured for causing a computer or computer network to fully or partially perform the method for determining at least one feature of at least one component of an analytical system according to any one of the preceding embodiments referring to a method for determining at least one feature of at least one component of an analytical system, when executed on the computer or computer network, wherein the computer program is configured to perform at least steps i) to iii) of the method for determining at least one feature of at least one component of an analytical system according to any one of the preceding embodiments referring to a method for determining at least one feature of at least one component of an analytical system.
Embodiment 17: A computer implemented method for monitoring and/or controlling at least one feature of at least one component of an analytical system, wherein in the method at least one system for monitoring and/or controlling according to any one of the preceding embodiments referring to a system for monitoring and/or controlling is used, wherein the method comprises the following steps:
   I) receiving a plurality of events via the first communication interface of the cloud-based infrastructure;
   II) storing the received events with the storage unit of the cloud-based infrastructure;
   III) streaming events to the event chain reaction system via the streaming platform;
   IV) detecting the feature of the component of the analytical system by matching the chain to the event stream with the event chain reaction system;
   V) providing the at least one reaction triggered by the event chain reaction system to the downstream system via the second interface;
   VI) the downstream system performing at least one action based on the reaction, wherein the action is one or more of generating a notification about the feature to at least one operator and/or controlling the feature of the component of the analytical system by adapting at least one property of the component of the analytical system.
Embodiment 18: The method according to any one of the preceding embodiments referring to a method for monitoring and/or controlling, wherein the method comprises transforming the events into a generic data structure by using at least one extraction and storage unit of the cloud-based infrastructure.
Embodiment 19: The method according to any one of the preceding embodiments referring to a method for monitoring and/or controlling, wherein the method comprises exchange of data between components of the cloud-based infrastructure via at least one notification bus.
Embodiment 20: Computer program for monitoring and/or controlling at least one feature of at least one component of an analytical system, configured for causing a computer or computer network to fully or partially perform the method for monitoring and/or controlling at least one feature of at least one component of an analytical system according to any one of the preceding embodiments referring to a method for monitoring and/or controlling at least one feature of at least one component of an analytical system, when executed on the computer or computer network, wherein the computer program is configured to perform at least steps I) to VI) of the method for monitoring and/or controlling at least one feature of at least one component of an analytical system according to any one of the preceding embodiments referring to a method for monitoring and/or controlling at least one feature of at least one component of an analytical system.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figure 1: shows an embodiment of a system for monitoring and/or controlling at least one feature of at least one component of an analytical system;
- Figure 2: shows another embodiment of a system for monitoring and/or controlling at least one feature of at least one component of an analytical system;
- Figure 3: shows an embodiment of a chain reaction system;
- Figure 4: shows an embodiment of a chain reaction component;
- Figure 5: shows a chain matching process in a chain matching element;
- Figure 6: shows a no chain matching process in a chain matching element;
- Figure 7: shows a flow chart of an embodiment computer implemented method for determining at least one feature of at least one component of an analytical system; and
- Figure 8: shows a flow chart of an embodiment of computer implemented method for monitoring and/or controlling at least one feature of at least one component of an analytical system.

### Detailed description of the embodiments

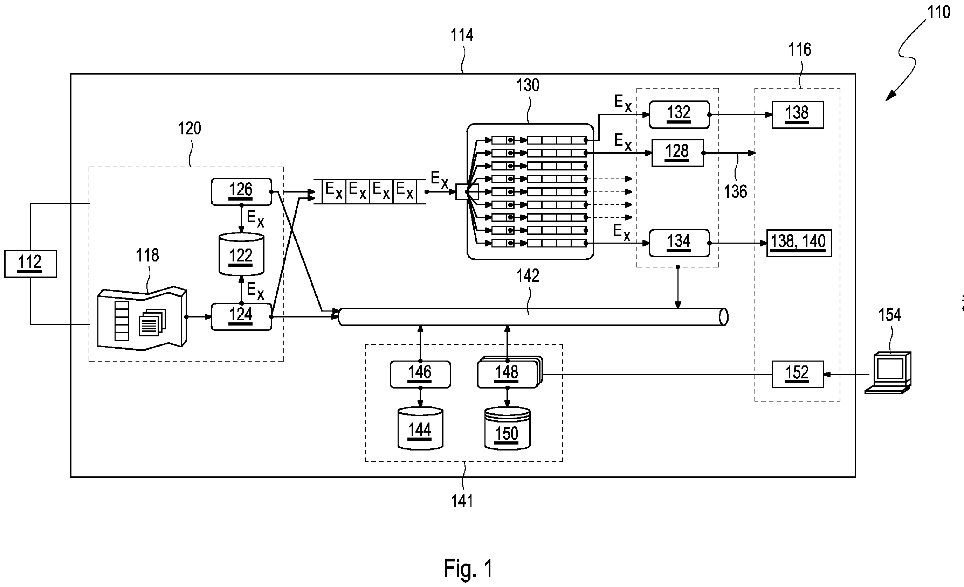

In Figure 1, an exemplary embodiment of a system 110 for monitoring and/or controlling at least one feature of at least one component of an analytical system 112 is shown in a schematic view. The system 110 for monitoring and/or controlling comprises at least one cloud-based infrastructure 114 and at least one downstream system 116.

The analytical system 112 may publish or stream events to the cloud-based infrastructure 114. Additionally or alternatively, the analytical system 112 may generate at least one file containing a data collection 118, such as a batch of data, specifically containing a plurality of events.

The at least one cloud-based infrastructure 114 comprises at least one first communication interface 120 for receiving the plurality of events. Specifically, the first communication interface 120 may receive the plurality of events from the analytical system 112 contained in the at least one file and/or in at least one event stream. The cloud-based infrastructure 114 further comprises at least one storage unit 122. The storage unit 122 is configured for storing the received events. Thus, the at least one communication interface 120 may be configured for extracting events 124 from the at least one file containing the data collection 118 from the analytical system 112 and, further, for transmitting the received events to the at least one storage unit 122. In case the analytical system 112 publishes or streams the plurality of events directly to the cloud-based infrastructure 114, the first communication interface 120 may be configured for storing the events 126 of the event stream separately, such as one by one, on the storage unit 122.

The cloud-based interface infrastructure 114 further comprises at least one event chain reaction system 128 and at least one streaming platform 130. The at least one streaming platform 130 is configured for streaming events to the event chain reaction system 128. The event chain reaction system 128 is configured for detecting the feature of the component of the analytical system 112 by matching the chain to the event stream.

The cloud-based infrastructure 114 may comprise further components connected to the at least one streaming platform 130. For example, the cloud-based infrastructure 114 may comprise at least one data aggregator 132 which may be configured for aggregating data received from the streaming platform 130 via the event stream. The cloud-based infrastructure 114 may also comprise at least one customer data aggregator 134. The customer data aggregator 134 may be configured for receiving events of the event stream from the streaming platform 130 and, further, for aggregating the received data. The cloud-based infrastructure 114 may allow a parallel event processing of the event stream by the event chain reaction system 128 and the further components, such as the data aggregator 132 and/or the customer data aggregator 134.

Further, the cloud-based infrastructure 114 comprises at least one second interface 136 which is configured for providing the at least one reaction triggered by the event chain reaction system 128 to the downstream system 116. The at least one downstream system 116 is configured for performing at least one action based on the reaction. The action is one or more of generating a notification about the feature to at least one operator and/or controlling the feature of the component of the analytical system 112 by adapting at least one property of the component of the analytical system 112.

The downstream system 116 may further comprise at least one platform 138, such as at least one platform 140. The at least one platform 138 may be connected to the at least one data aggregator 132, specifically to the at least one customer data aggregator 134, and may be configured for receiving the aggregated data.

Further, the cloud-based infrastructure 114 may comprise at least one extraction and storage unit 141. The at least one extraction and storage unit 141 may be configured for transforming the events into a generic structure.

The cloud-based infrastructure 114 may further comprise at least one notification bus 142. The notification bus 142 may be configured for transferring data, specifically events from the event stream, between one or more components of the cloud-based infrastructure 114. Specifically, the notification bus 142 may be configured for transferring system-level events between one or more components of the cloud-based infrastructure 114. For example, the notification bus 142 may be configured for transferring events from the first communication interface 120 to the at least one extraction and storage unit 141.

The at least one extraction and storage unit 141 may comprise further components. Specifically, the extraction and storage unit 141 may comprise at least one system event store 144. The system event store 144 may be connected to the notification bus 142 via at least one system event processor 146. The system event processor 146 may subscribe to the notification bus 142 and, thus, may be configured for storing the events of the event stream on the system event store 144. The system event processor 146 may also be comprised by the extraction and storage unit 141.

Further, the extraction and storage unit 141 may comprise at least one database management system 148. The databased management system 148 may subscribe to the at least one notification bus 142. Further, the database management system 148 may be configured for reading models in the events from the event stream received via the notification bus 142 and for storing the events of the event stream in at least one relational database 150, which may further be comprised by the extraction and storage unit 141. The database management system 148 may be accessible via the at least one downstream system 116. Specifically, the downstream system 116 may comprise at least one application programming interface (API) 152, such as a UI API. The application programming interface 152 may configured for reading data from the relational database 150 via the database management system 148. Further, the application programming interface 152 may be connected to at least one web browser 154 via at least one network and, thus, may be accessible via the at least one network using a computer outside the cloud-based infrastructure 114.

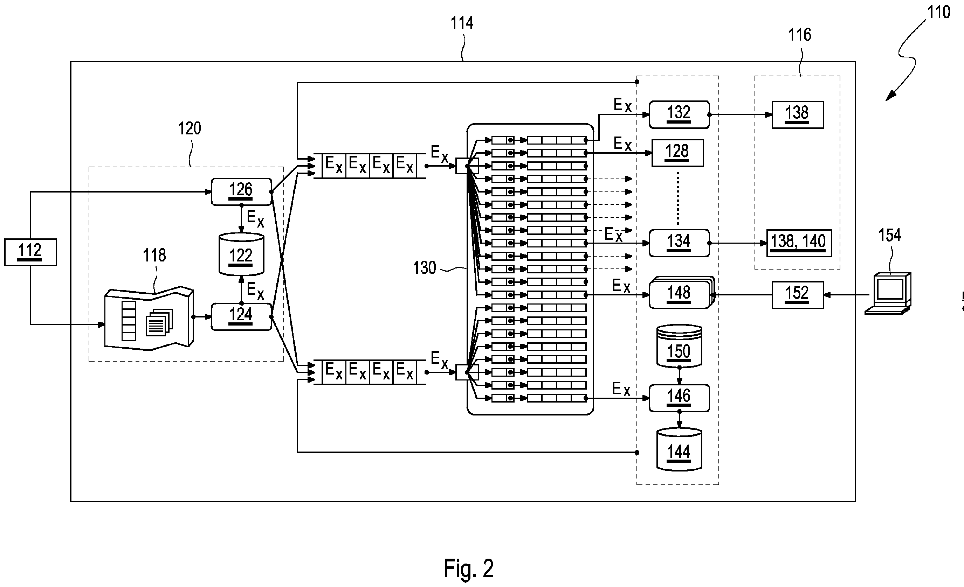

In Figure 2, another exemplary embodiment of a system 110 for monitoring and/or controlling at least one feature of at least one component of an analytical system 112 is shown in a schematic view. The system 110 shown in Figure 2 may correspond widely to the system 110 shown in Figure 1. Thus, reference may be made to the description of Figure 1.

In contrast to the system 110 shown in Figure 1, the system 110 of Figure 2 may not comprise a notification bus 142. Instead of the notification bus 142, the at least one streaming platform 130 may be used for transferring system-level notifications between one or more components of the system 110. Thus, the parallel event processing may further comprise the processing of events of the event stream by the system event store 144 and/or by the database management system 148. Thus, one or more or even all of the event chain reaction system 128, the data aggregator 132, the customer data aggregator 134, the system event store 144 and/or the database management system 148 may publish events, specifically system-level events, to the at least one streaming platform 130.

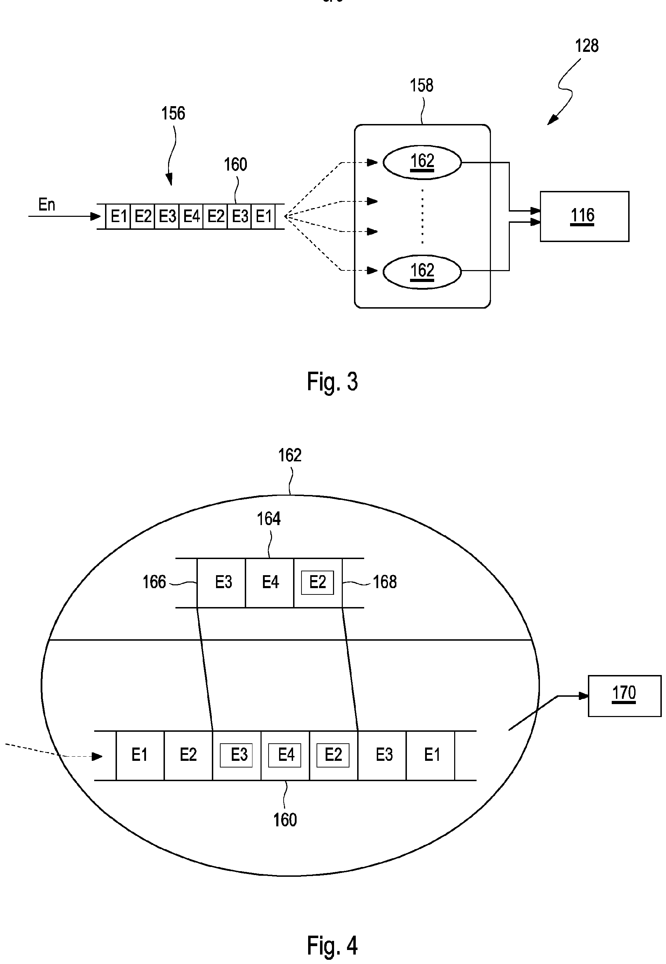

In Figure 3, an exemplary embodiment of an event chain reaction system 128 is shown in a schematic view. The event chain reaction system 128 comprises at least one communication interface 156 and at least one chain reaction component 158. The at least one chain reaction component 158 comprises at least one chain matching element 162.

The at least one communication interface 156 is configured for receiving at least one event stream 160. The event stream 160 comprises at least one sequence of ordered events generated by the at least one analytical system 112. Further, each event comprises information about a change in a state of the analytical system 112 and/or any of loaded resources. The at least one communication interface 156 may be configured for transmitting the events of the event stream 160 to the chain reaction component 158, specifically to the at least one chain matching element 162. In particular, the events of the event stream 160 may be sent separately, such as one by one, to the chain reaction component 158.

The chain matching element 162 is configured for recognizing at least one chain on the event stream 160, wherein the chain comprises a set of ordered events to be searched. A first event of the chain defines a start event 166. The chain matching element 162 is configured for identifying the start event 166 in the event stream 160 and, upon identifying the start event 166, the chain matching element 162 is configured for successively determining whether the other events of the chain match to one of the events of the event stream 160. In case all events of the chain are matched to events of the event stream 160, the chain matching element 162 is configured for triggering at least one reaction. The reaction comprises generating information that a chain was matched and/or issuing a command to at least one component of the analytical system 112. The chain matching element 162 may further be configured for providing the at least one reaction to the downstream system 116 of the cloud-based infrastructure 114. In case one of the events of the chain is not matched, the chain matching element 162 is configured for resetting to its initial state and waiting for the start event 166.

Figure 4 shows an exemplary embodiment of a chain matching element 162 in a schematic view. The chain matching element 162 may be configured for receiving the at least one event stream 160, as indicated in Figure 4 by the arrow pointing to the chain matching element 162. The chain matching element 162 may store at least one event chain 164 to be searched. Specifically, each chain matching element 162 may store one event chain 164 to be searched. Further, the chain matching element 162 may wait until the event stream 160 comprises the start event 166 of the event chain 164. Thus, after identifying the start event 166, the chain matching element 162 may determine if the sequence of events on the event stream 160 may correspond to the sequence of events of the event chain 164. The end of the event chain 164 may be marked by an end event 168. If the sequence of events on the event stream 160 matches the sequence of events of the event chain 164 between the start event 166 and the end event 168, the chain matching element 162 may be configured for triggering a reaction that indicates the match 170.

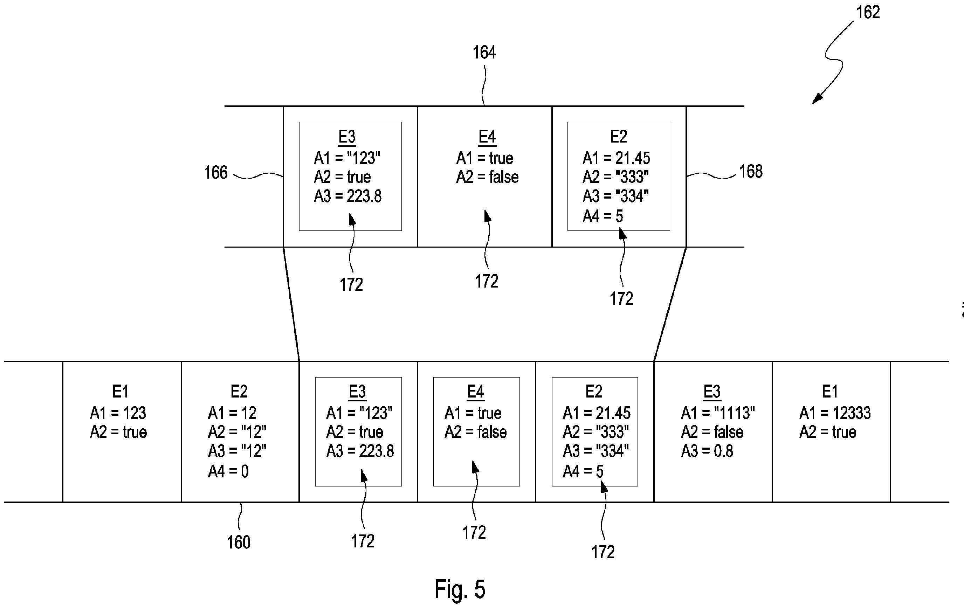

In Figure 5, a chain matching process in a chain matching element 162 is shown. The chain matching component 162 may comprise an event chain 164 to be searched. The event chain 164 may comprise a sequence of events, wherein the events of the event chain 164 may also comprise one or more attributes 172. The chain matching element 162 may start to compare the event chain 164 with the event stream 160 if the start event 166 was found on the event stream 160. The chain matching element 162 may further compare the events of the event chain 164 to be searched with the events of the event stream 160, also taking into account the attributes 172 of the respective event. In the exemplary event stream 160 shown in Figure 5, the event stream 160 matches the event chain 164 to be searched: The expected sequence of events of the event chain 164 and their attributes 172 is found in the event stream 160. Consequently, the event chain 164 of the chain matching element 162 may be considered to be matched and a reaction may be triggered by the chain matching element 162 indicating the match.

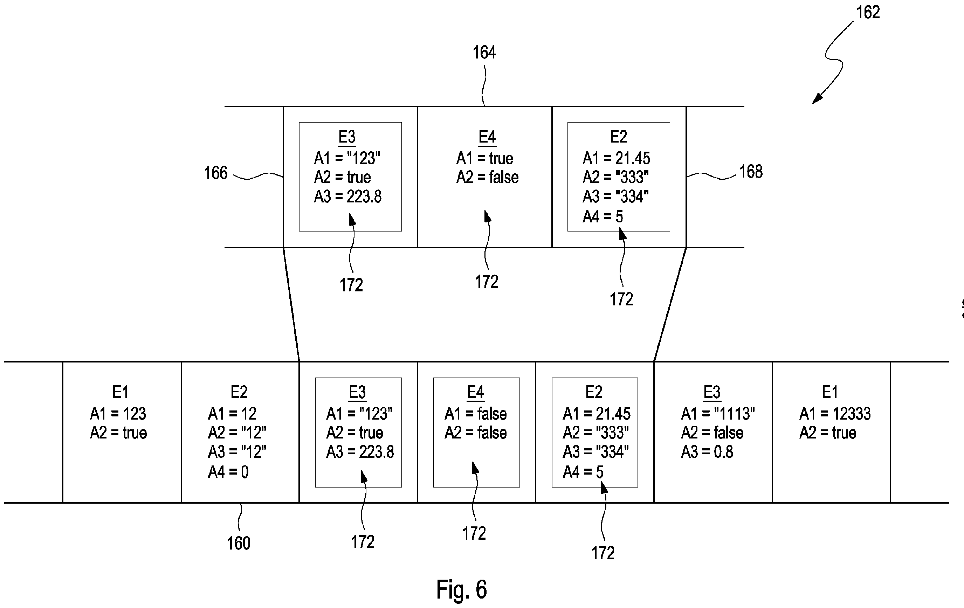

In Figure 6, a no chain matching process in a chain matching element 162 is shown. The chain matching element 162 may compare the event chain 164 with the received event stream 160. In the example shown in Figure 6, the start event 166 is matched to an event of the event stream 160, but the second event has a problem: The type of the expected event matches the type of the received event on the event stream 160 and, thus, the type of the second event may be considered correct. Contrarily, the attributes 172 of the received events is different from the expected event of the event chain 164. In this example, the attribute 172 A1 of the second event on the event chain 164 has the expected value of true but the received event of the event stream 160 has an A1 attribute value of false. In this example, the event chain 164 may be considered broken and, thus, no match nor reaction may be triggered by the chain matching element 162. Once the event chain 164 is considered broken, the chain matching element 162 may reset itself and may start waiting for the start event 166 again.

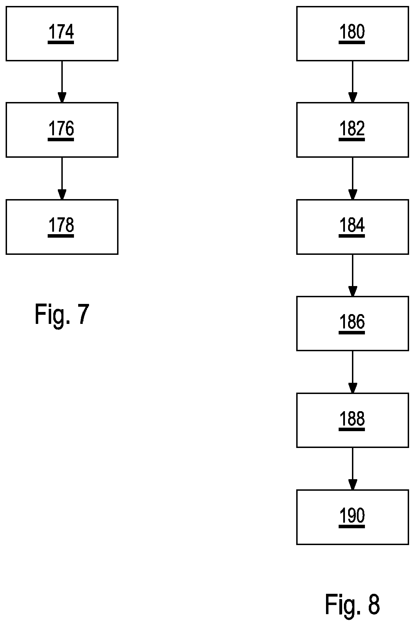

In Figure 7, a flow chart of an exemplary embodiment of a computer implemented method for determining at least one feature of at least one component of an analytical system 112 is shown. At least one event chain reaction system 128 is used in the method. Further, the method comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps that are not listed.

The method comprises:
i) (denoted by reference number 174) providing at least one event stream 160 via the communication interface 156, wherein the event stream 160 comprises at least one sequence of ordered events generated by the component of the analytical system 112, wherein each event comprises information about a change in a state of the analytical system 112 and/or any of the loaded resources;
ii) (denoted by reference number 176) determining the feature of the component of the analytical system 112 by recognizing at least one chain on the event stream 160 by using the chain matching element 162 of the chain reaction component 158, wherein the chain comprises a set of ordered events to be searched, wherein a first event of the chain defines a start event 166, wherein the recognizing comprises identifying the start event 166 in the event stream 160 and, upon identifying the start event 166, successively determining whether the other events of the chain match to one of the events of the event stream 160;
iii) (denoted by reference number 178) triggering at least one reaction, in case all events of the chain are matched to events of the event stream 160, wherein the reaction comprises generating information that a chain was matched and/or issuing a command to at least one component of the analytical system 112, or resetting of the chain matching element 162 to its initial state and waiting for the start event 166, in case one of the events of the chain is not matched.

Figure 8 shows a flow chart of an exemplary embodiment of a computer implemented method for monitoring and/or controlling at least one feature of at least one component of an analytical system 112. In the method at least one system 110 for monitoring and/or controlling is used. Further, the method comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method may comprise additional method steps that are not listed.

The method comprises the following steps:
I) (denoted by reference number 180) receiving a plurality of events via the first communication interface 120 of the cloud-based infrastructure 114;
II) (denoted by reference number 182) storing the received events with the storage unit 122 of the cloud-based infrastructure 114;
III) (denoted by reference number 184) streaming events to the event chain reaction system 128 via the streaming platform 130;
IV) (denoted by reference number 186) detecting the feature of the component of the analytical system 112 by matching the chain to the event stream 160 with the event chain reaction system 128;
V) (denoted by reference number 188) providing the at least one reaction triggered by the event chain reaction system 128 to the downstream system 116 via the second interface 136;
VI) (denoted by reference number 190) the downstream system 116 performing at least one action based on the reaction, wherein the action is one or more of generating a notification about the feature to at least one operator and/or controlling the feature of the component of the analytical system 112 by adapting at least one property of the component of the analytical system 112.

### List of reference numbers

- 110: system for monitoring and/or controlling
- 112: analytical system
- 114: cloud-based infrastructure
- 116: downstream system
- 118: data collection
- 120: first communication interface
- 122: storage unit
- 124: extracting events
- 126: storing events
- 128: event chain reaction system
- 130: streaming platform
- 132: data aggregator
- 134: customer data aggregator
- 136: second interface
- 138: platform
- 140: platform
- 141: extraction and storage unit
- 142: notification bus
- 144: system event store
- 146: system event processor
- 148: database management system
- 150: relational database
- 152: application programming interface
- 154: web browser
- 156: communication interface
- 158: chain reaction component
- 160: event stream
- 162: chain matching element
- 164: event chain
- 166: start event
- 168: end event
- 170: match
- 172: attribute
- 174: providing at least one event stream
- 176: determining the feature of the component of the analytical system
- 178: triggering at least one reaction
- 180: receiving a plurality of events
- 182: storing the received events
- 184: streaming events
- 186: detecting the feature of the component of the analytical system
- 188: providing at least one reaction
- 190: performing at least one action

## Claims

1. An event chain reaction system (128) comprising
- at least one communication interface (156) configured for receiving at least one event stream (160), wherein the event stream (160) comprises at least one sequence of ordered events generated by at least one analytical system (112), wherein each event comprises information about a change in a state of the analytical system (112) and/or any of loaded resources;
- at least one chain reaction component (158) comprising at least one chain matching element (162), wherein the chain matching element (162) is configured for recognizing at least one chain on the event stream (160), wherein the chain comprises a set of ordered events to be searched, wherein a first event of the chain defines a start event (166), wherein the chain matching element (162) is configured for identifying the start event (166) in the event stream (160) and, upon identifying the start event (166), the chain matching element (162) is configured for successively determining whether the other events of the chain match to one of the events of the event stream (160), wherein, in case all events of the chain are matched to events of the event stream (160), the chain matching element (162) is configured for triggering at least one reaction, wherein the reaction comprises generating information that a chain was matched and/or issuing a command to at least one component of the analytical system (112), wherein, in case one of the events of the chain is not matched, the chain matching element (162) is configured for resetting to its initial state and waiting for the start event (166).

2. The event chain reaction system (128) according to the preceding claim, wherein the chain reaction component (158) comprises a plurality of chain matching elements (162), wherein the chain reaction component (158) is configured for multiple chain matching in parallel.

3. The event chain reaction system (128) according to the preceding claim, wherein the chain reaction component (158) is configured for triggering multiple reactions at the same time.

4. The event chain reaction system (128) according to any one of the preceding claims, wherein each of the event comprises a time stamp when the event was produced.

5. The event chain reaction system (128) according to any one of the preceding claims, wherein each of the event comprises an originator identifier indicating which identity has produced the event.

6. The event chain reaction system (128) according to any one of the preceding claims, wherein the chain is an ordered set of events indicative for a feature of the analytical system (112).

7. The event chain reaction system (128) according to any one of the preceding claims, wherein the information about a change in a state of the analytical system (112) is at least one of an operational information or a sensor signal generated by at least one sensor of the analytical system (112).

8. The event chain reaction system (128) according to the preceding claim, wherein the information about a change in a state of the analytical system (112) is at least one information selected from the group consisting of: a sensor signal indicating that performance is changing; a sensor signal indicating that a sample rack is loaded; a sensor signal indicating that sample container is identified, a sensor signal indicating that over temperature is detected, a sensor signal indicating that a fuse is broken, a sensor signal indicating that performance is changing, a sensor signal indicating that at least one hardware component of the analytical system (112) performs differently as expected.

9. A system (110) for monitoring and/or controlling at least one feature of at least one component of an analytical system (112), wherein the system comprises at least one cloud-based infrastructure (114), wherein the cloud-based infrastructure (114) comprises at least one first communication interface (120) configured for receiving a plurality of events, wherein the cloud-based infrastructure (114) comprises at least one storage unit (122) configured for storing the received events, wherein the cloud-based infrastructure (114) comprises at least one event chain reaction system (128) according to any one of the preceding claims, wherein the cloud-based infrastructure (124) comprises at least one streaming platform (130) configured for streaming events to the event chain reaction system (128), wherein the event chain reaction system (128) is configured for detecting the feature of the component of the analytical system (112) by matching the chain to the event stream (160), wherein the system (110) comprises at least one downstream system (116), wherein the cloud-based infrastructure (114) comprises at least one second interface (136) which is configured for providing the at least one reaction triggered by the event chain reaction system (128) to the downstream system (116), wherein the downstream system (116) is configured for performing at least one action based on the reaction, wherein the action is one or more of generating a notification about the feature to at least one operator and/or controlling the feature of the component of the analytical system (112) by adapting at least one property of the component of the analytical system (112).

10. The system (110) for monitoring and/or controlling according to the preceding claim, wherein the streaming platform (130) is configured for streaming events to a plurality of subscribers in parallel.

11. The system (110) for monitoring and/or controlling according to any one of the two preceding claims, wherein the cloud-based infrastructure (114) comprises at least one extraction and storage unit (141) configured for transforming the events into a generic data structure.

12. The system (110) for monitoring and/or controlling according to any one of the three preceding claims, wherein the cloud-based infrastructure (114) comprises at least one notification bus (142) configured for exchange data between components of the cloud-based infrastructure (114).

13. The system (110) for monitoring and/or controlling according to any one of the four preceding claims, wherein the system (110) comprises at least one prediction unit configured for predicting future behavior of the analytical system based on event data.

14. A computer implemented method for determining at least one feature of at least one component of an analytical system (112), wherein in the method at least one event chain reaction system (128) according to any one of the preceding claims referring to an event chain reaction system (128) is used, wherein the method comprises the following steps:
i) providing at least one event stream (160) via the communication interface (156), wherein the event stream (160) comprises at least one sequence of ordered events generated by the component of the analytical system (112), wherein each event comprises information about a change in a state of the analytical system (112) and/or any of the loaded resources;
ii) determining the feature of the component of the analytical system (112) by recognizing at least one chain on the event stream (160) by using the chain matching element (162) of the chain reaction component (158), wherein the chain comprises a set of ordered events to be searched, wherein a first event of the chain defines a start event (166), wherein the recognizing comprises identifying the start event (166) in the event stream (160) and, upon identifying the start event (166), successively determining whether the other events of the chain match to one of the events of the event stream (160);
iii)triggering at least one reaction, in case all events of the chain are matched to events of the event stream (160), wherein the reaction comprises generating information that a chain was matched and/or issuing a command to at least one component of the analytical system (112), or resetting of the chain matching element (162) to its initial state and waiting for the start event (166), in case one of the events of the chain is not matched.

15. The method according to the preceding claim, wherein the chain is an ordered set of events indicative for a feature of the analytical system (112).

16. Computer program for determining at least one feature of at least one component of an analytical system (112), configured for causing a computer or computer network to fully or partially perform the method for determining at least one feature of at least one component of an analytical system (112) according to any one of the preceding claims referring to a method for determining at least one feature of at least one component of an analytical system (112), when executed on the computer or computer network, wherein the computer program is configured to perform at least steps i) to iii) of the method for determining at least one feature of at least one component of an analytical system (112) according to any one of the preceding claims referring to a method for determining at least one feature of at least one component of an analytical system (112).

17. A computer implemented method for monitoring and/or controlling at least one feature of at least one component of an analytical system (112), wherein in the method at least one system (110) for monitoring and/or controlling according to any one of the preceding claims referring to a system (110) for monitoring and/or controlling is used, wherein the method comprises the following steps:
I) receiving a plurality of events via the first communication interface (120) of the cloud-based infrastructure (114);
II) storing the received events with the storage unit (122) of the cloud-based infrastructure (114);
III) streaming events to the event chain reaction system (128) via the streaming platform (130);
IV) detecting the feature of the component of the analytical system (112) by matching the chain to the event stream (160) with the event chain reaction system (128);
V) providing the at least one reaction triggered by the event chain reaction system (128) to the downstream system (116) via the second interface (136);
VI) the downstream system (116) performing at least one action based on the reaction, wherein the action is one or more of generating a notification about the feature to at least one operator and/or controlling the feature of the component of the analytical system (112) by adapting at least one property of the component of the analytical system (112).

18. The method according to any one of the preceding claims referring to a method for monitoring and/or controlling, wherein the method comprises transforming the events into a generic data structure by using at least one extraction and storage unit (141) of the cloud-based infrastructure (114).

19. The method according to any one of the preceding claims referring to a method for monitoring and/or controlling, wherein the method comprises exchange of data between components of the cloud-based infrastructure (114) via at least one notification bus (142).

20. Computer program for monitoring and/or controlling at least one feature of at least one component of an analytical system (112), configured for causing a computer or computer network to fully or partially perform the method for monitoring and/or controlling at least one feature of at least one component of an analytical system (112) according to any one of the preceding claims referring to a method for monitoring and/or controlling at least one feature of at least one component of an analytical system (112), when executed on the computer or computer network, wherein the computer program is configured to perform at least steps I) to VI) of the method for monitoring and/or controlling at least one feature of at least one component of an analytical system (112) according to any one of the preceding claims referring to a method for monitoring and/or controlling at least one feature of at least one component of an analytical system (112).
